(19) Europäisches Patentamt
European Patent Office
Office européen des brevets

(11) **EP 3 392 299 A1**

(12) **EUROPEAN PATENT APPLICATION**
published in accordance with Art. 153(4) EPC

(43) Date of publication:
24.10.2018 Bulletin 2018/43

(51) Int Cl.:
*C08J 9/28* (2006.01)     *B01J 19/00* (2006.01)
*B29C 39/24* (2006.01)     *C12M 3/00* (2006.01)
*G02B 3/00* (2006.01)     *B29K 105/04* (2006.01)

(21) Application number: 16875587.4

(22) Date of filing: 12.12.2016

(86) International application number:
PCT/JP2016/086905

(87) International publication number:
WO 2017/104610 (22.06.2017 Gazette 2017/25)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 14.12.2015   JP 2015243016
20.07.2016   JP 2016142659

(71) Applicant: Japan Science and Technology Agency
Kawaguchi-shi
Saitama 332-0012 (JP)

(72) Inventors:
• IWAI Yosuke
Suita-shi
Osaka 565-0871 (JP)
• UCHIDA Yoshiaki
Suita-shi
Osaka 565-0871 (JP)
• YABU Hiroshi
Sendai-shi
Miyagi 980-8577 (JP)

(74) Representative: Cabinet Plasseraud
66, rue de la Chaussée d'Antin
75440 Paris Cedex 09 (FR)

(54) **POROUS FILM, METHOD FOR MANUFACTURING POROUS FILM, MICROLENS ARRAY, MICROREACTOR, AND     BIO-DEVICE**

(57)     A method for manufacturing a porous film includes: a first step of preparing droplets (D) which are formed from a first liquid into spheres with a predetermined diameter of 10 μm or more and 2000 μm or less and a second liquid (L2) which includes a curing agent which cures by imparting energy or a curing agent which cures due to change in pH and includes droplets dispersed therein; a second step of injecting the droplets and the second liquid into a gap between a pair of substrates (31 and 32); a third step of curing the second liquid to form an external phase; and the fourth step of removing the droplets in the external phase to form hole sections.

FIG. 6

EP 3 392 299 A1

**Description**

[Technical Field]

The present invention relates to a porous film, a method for manufacturing a porous film, a microlens array, a microreactor, and a bio-device.
Priority is claimed on Japanese Patent Application Nos. 2015-243016 and 2016-142659, filed December 14, 2015 and July 20, 2016, respectively, the content of which is incorporated herein by reference.

[Background Art]

Porous films are manufactured by combining various polymer materials and porosity forming technologies and are expected to be able to be applied to microlens arrays, cell culture substrates, and anti-adhesion films. Particularly, porous films with a hole diameter of about 100 to 1000 $\mu$m and a uniform hole diameter have attracted attention because these porous films can be applied to microlenses when substances with different refractive indices are introduced into vacancies thereof or can selectively separate or support substances in this size range.
Examples of a method for manufacturing this kind of porous film include laser processing or photolithography using an optical device (for example, refer to Non-Patent Document 1), a top-down method such as cutting using a drill (for example, refer to Non-Patent Document 2), or a bottom-up method using self-organization such as a solvent casting method (for example, refer to Non-Patent Document 3). Manufacturing methods using the laser processing or photolithography are processes which basically merely perform two-dimensional patterning and incur high manufacturing costs when holes in this size range are processed with a larger area. Furthermore, in fine hole processing using a drill, there is a problem that it is difficult to finish the burrs generated on an outlet side.
On the other hand, the method using self-organization can perform mass production at low cost compared with the laser processing, photolithography, or the like using the optical device. Furthermore, Patent Document 1 describes a technique of forming condensation on a casting film to form water droplets and then evaporating a solvent and the water droplets from the casting film to form a porous film.

[Citation List]

[Patent Literature]

[Patent Document 1] Japanese Unexamined Patent Application, First Publication No. 2014-156526

[Non-Patent Documents]

[Non-Patent Document 1] H. J. Lee et al., Acta Biomaterialia 2011, 7, 1281 to 1289.
[Non-Patent Document 2] P. Yilgor et al., J. Tissue Eng. Regen. Med. 2013, 7, 687 to 696.
[Non-Patent Document 3] H. Yabu et al., Macromolecules 2011, 44, 5868 to 5873.

[Summary of Invention]

[Technical Problem]

However, in the method using self-organization such as the solvent casting method described above or the method described in Patent Document 1, fine holes of about 100 nm to 20 $\mu$m can be formed, but holes with a hole diameter of about 100 to 1000 $\mu$m cannot be formed. Furthermore, it can be expected that a film with a hole diameter of about 100 to 1000 $\mu$m having not only two-dimensional but also three-dimensional porous structures would be able to be applied as a scaffolding material capable of allowing adhesion and proliferation of cells of several to several tens of $\mu$m in the holes. Therefore, it is required that a porous film with a hole diameter of about 10 to 2000 $\mu$m be able to be manufactured from various types of material at low cost.
The present invention was made in view of the above-described circumstances, and an object of the present invention is to provide a porous film, a method for manufacturing a porous film, a microlens array, a microreactor, and a bio-device capable of manufacturing a porous film having hole sections with a diameter of 10 $\mu$m or more and 2000 $\mu$m or less at low cost.

[Solution to Problem]

The inventors of the present invention carried out intensive research, and as a result, found the excellent effect that, when a first step of preparing droplets which are formed from a first liquid into spheres with a predetermined diameter of 10 μm or more and 2000 μm or less and a second liquid which includes a photocuring agent and includes the droplets dispersed therein, a second step of injecting the droplets and the second liquid into a gap between a pair of substrates having optical transparency, a third step of curing the second liquid by irradiation with light through the substrates to form an external phase, and a fourth step of removing the droplets in the external phase to form hole sections are provided, a high quality porous film which have hole sections with a diameter of 10 μm or more and 2000 μm or less and have low manufacturing costs and a method for manufacturing the porous film can be provided and have completed the present invention.

That is to say, in order to solve the above object, the present invention adopts the following means.

(1) A first step of preparing droplets which are formed from a first liquid into spheres with a predetermined diameter of 10 μm or more and 2000 μm or less and a second liquid which includes a curing agent which cures by imparting energy or a curing agent which cures due to change in pH and includes the droplets dispersed therein; a second step of injecting the droplets and the second liquid into a gap between a pair of substrates; a third step of curing the second liquid to form a base; and a fourth step of removing the droplets in the base to form hole sections are provided.

(2) In the method for manufacturing a porous film according to (1), the second liquid may include a surfactant and a stabilizer.

(3) In the method for manufacturing a porous film according to (1) or (2), the energy may be light or heat.

(4) In the method for manufacturing a porous film according to any one of (1) to (3), a specific gravity of the first liquid may be larger than a specific gravity of the second liquid.

(5) In the method for manufacturing a porous film according to any one of (1) to (4), the distance between the pair of substrates may be adjusted so that the droplets are arranged in one layer in the gap.

(6) In the method for manufacturing a porous film according to any one of (1) to (4), the droplets may be arranged in a plurality of layers in the gap.

(7) In the method for manufacturing a porous film according to (6), the droplets may be arranged in a body-centered cubic structure.

(8) In the method for manufacturing a porous film according to (6), the droplets may be arranged in a hexagonal close-packed structure.

In the method for manufacturing a porous film according to (6), the droplets may be arranged in a face-centered cubic structure.

In the method for manufacturing a porous film according to any one of (1) to (9), in the first step, droplets of a plurality of types with different diameters may be prepared.

In the method for manufacturing a porous film according to any one of (1) to (10), the first step may include a step of causing the first liquid to flow into a flow path of a first tube and causing the droplets of the first liquid to flow from a nozzle of the first tube into the second liquid flowing through a flow path of a second tube.

In the method for manufacturing a porous film according to (11), at least one of a relative rate of the first liquid flowing through the first tube with respect to a rate of the second liquid flowing through the second tube and a diameter of the nozzle may be adjusted depending on a diameter of the droplets to be prepared.

In the method for manufacturing a porous film according to any one of (1) to (12), at least one of the first liquid and the second liquid may be an oil phase and the other thereof may be an aqueous phase.

In the method for manufacturing a porous film according to any one of (1) to (13), the step of removing the droplets may include a step of cleaning the droplets.

In the method for manufacturing a porous film according to any one of (1) to (14), the droplets may be formed with a predetermined diameter of 250 μm or more and 2000 μm or less.

(16) A porous film according to an aspect of the present invention includes: a plurality of hole sections formed in a spherical shape with a predetermined diameter of 10 μm or more and 2000 μm or less; and a base including a curing agent which cures by imparting energy or a curing agent which cures due to change in pH, wherein the plurality of hole sections are arranged with a predetermined size and a relative error in a diameter thereof is 6% or less.

(17) In the porous film according to (16), the base may include a surfactant and a stabilizer.

(18) In the porous film according to (16) or (17), the hole sections arranged to be adjacent to each other may communicate with each other via communication holes.

(19) In the porous film according to any one of (16) to (18), the plurality of hole sections may be arranged in a body-centered cubic structure.

(20) In the porous film according to any one of (16) to (18), the plurality of hole sections may be arranged in a hexagonal close-packed structure.

(21) In the porous film according to any one of (16) to (18), the plurality of hole sections may be arranged in a face-centered cubic structure.

(22) In the porous film according to any one of (16) to (21), the plurality of hole sections may include a plurality of first hole sections formed with a first diameter and a plurality of second hole sections formed with a second diameter different from the first diameter, and the first hole sections and the second hole sections may be arranged in a predetermined regular manner and relative errors in diameters thereof are 6% or less.

(23) In the porous film according to any one of (16) to (22), the hole sections may be formed with a predetermined diameter of 250 $\mu$m or more and 2000 $\mu$m or less.

(24) A microlens array according to an aspect of the present invention includes: the porous film according to any one of (16) to (23); and lens bodies arranged in the hole sections.

(25) A microreactor according to an aspect of the present invention includes: the porous film according to any one of (16) to (23).

(26) A bio-device according to an aspect of the present invention includes: the porous film according to any one of (16) to (23).

[Advantageous Effects of Invention]

According to a method for manufacturing a porous film associated with an aspect of the present invention, a porous film having hole sections with a diameter of 10 $\mu$m or more and 2000 $\mu$m or less can be manufactured with low manufacturing costs and high quality.

According to a porous film associated with an aspect of the present invention, a high quality porous film having hole sections with a diameter of 10 $\mu$m or more and 2000 $\mu$m or less can be obtained with low manufacturing costs.

[Brief Description of Drawings]

Fig. 1 is a cross-sectional view of a porous film 1 formed between glass substrates 31 and 32 according to this embodiment.

Fig. 2 is a cross-sectional view taken along line A-A in Fig. 1.

Fig. 3 is a diagram showing a constitution of a first capillary device 40 used in a first step.

Fig. 4 is a diagram showing a constitution of a second capillary device 50 used in the first step.

Fig. 5 is a diagram showing a relationship among a flow rate of a first liquid L1, an average particle diameter of droplets D, and a relative error of a particle diameter.

Fig. 6 is an external perspective view showing an example of a device DV used in a second step.

Fig. 7 is a diagram showing a state in which the device DV is divided.

Fig. 8 is a diagram showing a process of manufacturing a porous film.

Fig. 9 is a plan view showing a model of oil droplets D arranged in two layers in a thickness direction.

Fig. 10 is a plan view showing a model of oil droplets D arranged in two layers in a thickness direction.

Fig. 11 is a diagram showing a state in which oil droplets D are arranged in a body-centered cubic structure.

Fig. 12 is a diagram showing a state in which oil droplets D are arranged in a hexagonal close-packed structure.

Fig. 13 is an enlarged view of a porous film 1 in which hole sections 10 are arranged in a body-centered cubic structure.

Fig. 14 is an enlarged view of a porous film 1 in which hole sections 10 are arranged in a hexagonal close-packed structure.

Fig. 15 is a front view of a model in which oil droplets D arranged in three layers are arranged in a body-centered cubic structure.

Fig. 16 is a plan view of a model in which oil droplets D arranged in three layers are arranged in a body-centered cubic structure.

Fig. 17 is a front view of a model in which oil droplets D arranged in three layers are arranged in a face-centered cubic structure.

Fig. 18 is a plan view of a model in which oil droplets D arranged in three layers are arranged in a face-centered cubic structure.

Fig. 19 is a diagram showing a state of arrangement in which there are two kinds of oil droplets D with a diameter ratio of about 2:3.

Fig. 20 is a diagram showing a state of arrangement in which there are two kinds of oil droplets D with a diameter ratio of about 1:2.

Fig. 21 is a diagram showing a state in which liquid crystal microcapsules have been captured in hole sections.

[Description of Embodiments]

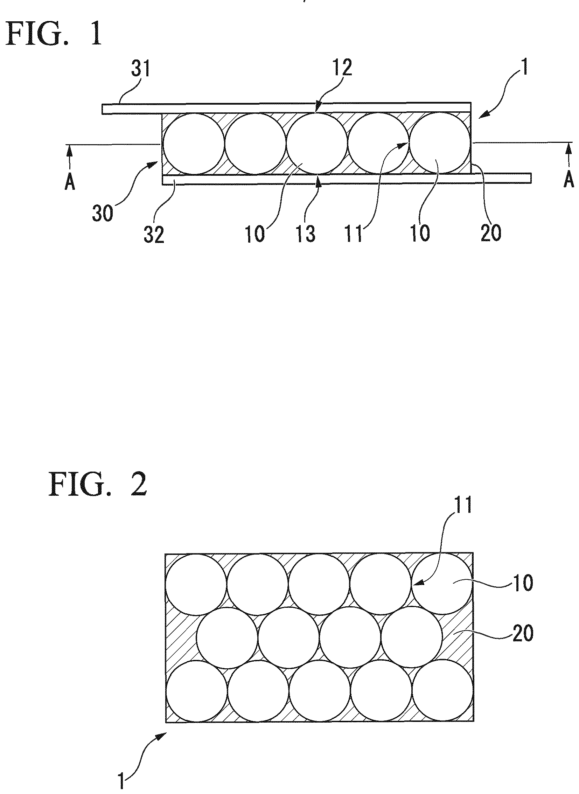

Embodiment modes of a porous film, a method for manufacturing a porous film, a microlens array, a microreactor, and a bio-device according to this embodiment will be described below with reference to Figs. 1 to 17.

(First Embodiment of Porous Film)

First, a porous film 1 in a first embodiment will be described with reference to Figs. 1 and 2.

Fig. 1 is a cross-sectional view of the porous film 1 formed between glass substrates (substrate) 31 and 32 arranged parallel to each other with a gap 30 therebetween. Fig. 2 is a cross-sectional view taken along line A-A in Fig. 1.

As shown in Figs. 1 and 2, the porous film 1 has a base 20 in which a plurality of hole sections 10 are arranged. For example, the base 20 may be made of a photocuring monomer which is cured by irradiating with light (imparting energy). For example, the base 20 may be made of monomers such as an acrylamide containing a photopolymerization initiator.

The hole sections 10 are formed in a spherical shape with a predetermined diameter and arranged in a single layer in a thickness direction of the porous film 1. A plurality of rows of the hole sections 10 whose center positions are aligned on a straight line are regularly arranged in a plane direction of the porous film 1. The hole sections 10 are arranged in a zigzag shape in which neighboring rows are shifted by distances of the radius (a half-pitch) of the hole sections 10. The diameter of the hole sections 10 is set to 10 $\mu$m or more and 2000 $\mu$m or less, preferably 250 $\mu$m or more and 2000 $\mu$m or less.

The hole sections 10 arranged adjacent to each other communicate with each other via communication holes 11. The hole sections 10 have holes 12 at joint portions between the hole sections 10 and the glass substrate 31. The hole sections 10 have holes 13 at joint portions between the hole sections 10 and the glass substrate 32.

(Method for Manufacturing Porous Film 1)

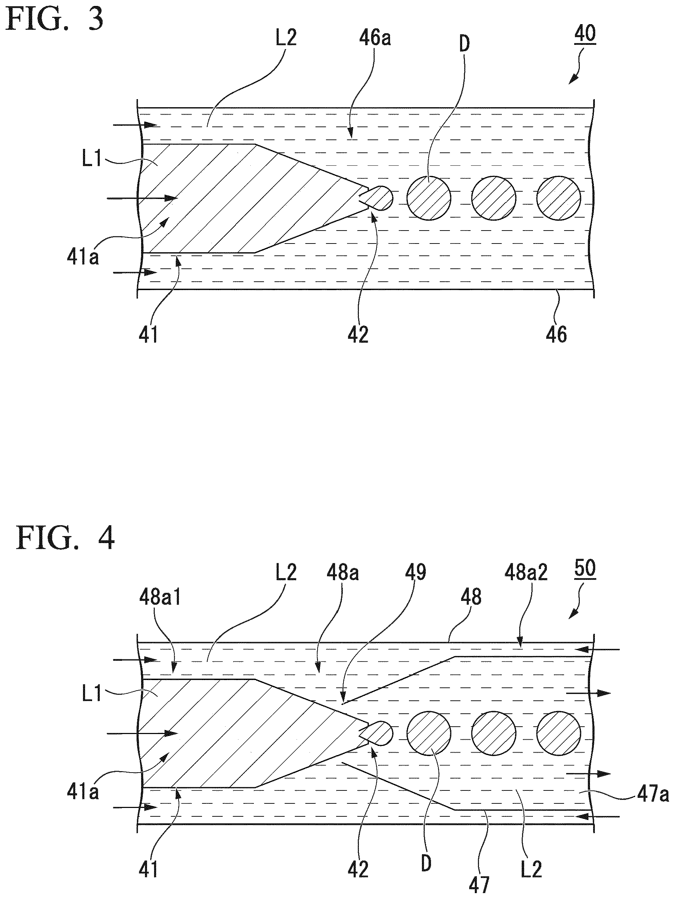

Next, a method for manufacturing the porous film 1 in the above constitution will be described with reference to Figs. 3 to 7. The method for manufacturing the porous film 1 in the above constitution includes a first step of preparing spherical droplets formed from a first liquid used for forming the hole sections 10 and a second liquid used for forming the base 20 and, a second step of injecting the spherical droplets and the second liquid into a gap between the glass substrates 31 and 32, a third step of curing the second liquid by irradiating the second liquid with light through the glass substrates 31 and 32 to form the base 20 to be an external phase, and a fourth step of removing the droplets in the base 20 to form the hole sections 10.

(First Step)

Fig. 3 is a diagram showing a constitution of a first capillary device 40 used in the first step. Fig. 4 is a diagram showing a constitution of a second capillary device 50 used in the first step. The first capillary device 40 and the second capillary device 50 are appropriately selected in accordance with a size of a diameter of each of droplets to be formed from the first liquid. Note that a diameter described in this embodiment is defined as an outer diameter of droplets observed with a microscope.

As shown in Fig. 3, the first capillary device 40 includes an injection tube (first tube) 41 through which a first liquid L1 flows into an internal flow path 41a and a recovery tube (second tube) 46 through which a second liquid L2 flows into an internal flow path 46a and into which the first tube 41 is inserted. The injection tube 41 and the recovery tube 46 are made of, for example, glass and arranged coaxially. A nozzle 42 for discharging the first liquid L1 is provided at a distal end of the injection tube 41. The flow path 41a and the flow path 46a extend in the same direction and the first liquid L1 and the second liquid L2 flow in the same direction (from the left side to the right side in Fig. 3).

As shown in Fig. 4, the second capillary device 50 includes the injection tube 41 described above, a recovery tube 47 through which the second liquid L2 flows into an internal flow path 47a and into which the nozzle 42 of the injection tube 41 is inserted, and an outer tube 48 in which the injection tube 41 and the recovery tube 47 are coaxially arranged in a flow path 48a. The recovery tube 47 and the outer tube 48 are made of, for example, glass. An end portion of the recovery tube 47 facing the injection tube 41 has an opening 49 with a larger diameter than an outer diameter of the nozzle 42 of the injection tube 41. The second liquid L2 is injected into a flow path 48a1 of the flow path 48a in the outer tube 48 which is between the injection tube 41 and the outer tube 48 in the same direction (from the left side to the right side in Fig. 3) as a flow direction of the first liquid L1. The second liquid L2 is injected into a flow path 48a2 of the flow path 48a in the outer tube 48 which is between the recovery tube 47 and the outer tube 48 in a direction (from the right side to the left side in Fig. 3) opposite to the flow direction of the first liquid L1. The second liquids L2 injected from the flow path 48a1 and the flow path 48a2 join and flows into the flow path 47a of the recovery tube 47 via the opening 49.

In the present embodiment, an oil phase liquid is used as the first liquid L1. For example, a liquid which includes polydimethylsiloxane and bromobenzene bromobenzene added as a stabilizer for oil droplets (with a volume ratio of 79/21) may be used as the first liquid L1.

An aqueous phase liquid is used as the second liquid L2. For example, a liquid containing water ($8.26 \times 10$ % by weight), an acrylamide (8.26 % by weight) as a monomer, N,N'-methylenebisacrylamide ($8.26 \times 10^{-1}$ % by weight) as a crosslinking agent, an alkylphenone-based photopolymerization initiator (IRGACURE (registered trademark) 2959) ($8.26 \times 10^{-2}$ % by weight) as a photopolymerization initiator, and polyvinyl alcohol (8.26 % by weight) as a surfactant may be used as the second liquid L2. In the present embodiment, an initiator which performs curing by imparting light energy as energy to be imparted is used. Water is degassed for 15 minutes, purged with nitrogen for 15 minutes, and then used.

In the present embodiment, a specific gravity of the first liquid L1 is larger than a specific gravity of the second liquid L2. Since the second liquid L2 in the embodiment is an aqueous phase and most of the mass thereof is water, the specific gravity thereof is substantially 1. For this reason, a material with a specific gravity larger than 1 is selected as the first liquid L1.

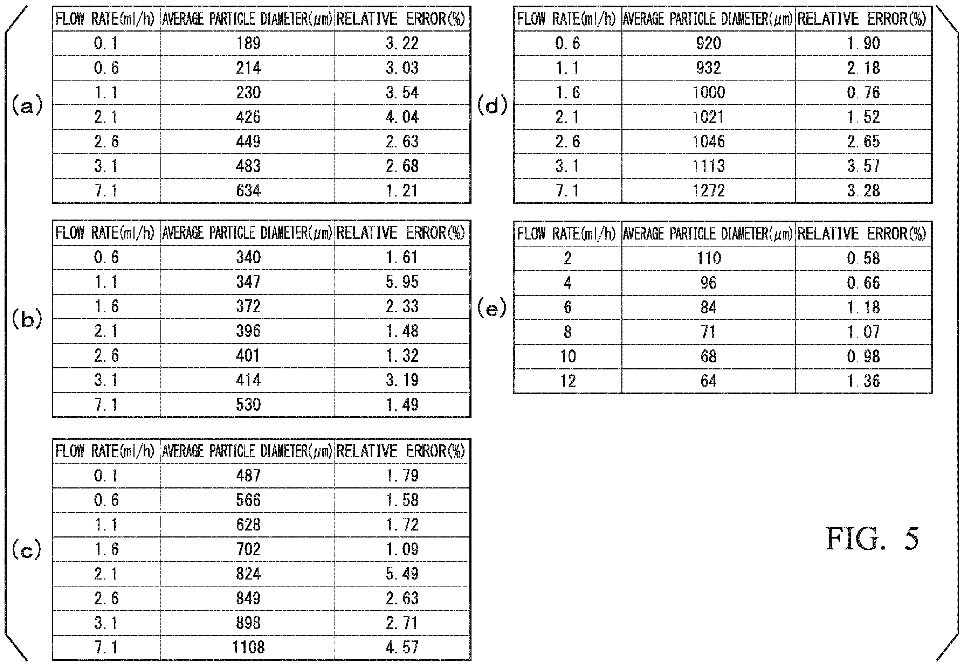

Fig. 5 is a diagram showing a relationship among a flow rate (ml/h) of a first liquid L1when both a flow rate of the second liquid L2 in the flow path 46a of the first capillary device 40 and a flow rate of the second liquid L2 in the flow path 48a1 and the flow path 48a2 of the second capillary device 50 are set to a fixed value of 11 ml/h, an average particle diameter (diameter; $\mu$m) of droplets D (hereinafter referred to as an "oil droplets D") formed when the first liquid L1 is caused to flow into a second liquid L2 in the flow path 46a or the flow path 47a, and a relative error (%) of a particle diameter .

(a) of Fig. 5 shows a relationship in a case in which a diameter (opening diameter) of the nozzle 42 in the injection tube 41 of the second capillary device 50 is 220 $\mu$m and a diameter (opening diameter) of the opening 49 in the recovery tube 47 is 440 $\mu$m. (b) of Fig. 5 shows a relationship of a case in which the diameter of the nozzle 42 in the injection tube 41 of the second capillary device 50 is 340 $\mu$m and the diameter of the opening 49 in the recovery tube 47 is 710 $\mu$m. (c) of Fig. 5 shows a relationship of a case in which a diameter of the nozzle 42 in the injection tube 41 of the first capillary device 40 is 170 $\mu$m. (d) of Fig. 5 shows a relationship of a case in which the diameter of the nozzle 42 in the injection tube 41 of the first capillary device 40 is 770 $\mu$m. (e) of Fig. 5 shows a relationship of a case in which the diameter (opening diameter) of the nozzle 42 in the injection tube 41 of the second capillary device 50 is 50 $\mu$m and the diameter (opening diameter) of the opening 49 in the recovery tube 47 is 120 $\mu$m.

As shown in (a) to (e) of Fig. 5, oil droplets D with particle diameters of 63 to 1272 $\mu$m is prepared by selecting the first capillary device 40 or the second capillary device 50 and appropriately selecting a diameter of the nozzle 42, a diameter of the opening 49, and a flow rate (a relative rate to a flow rate of the second liquid L2) of the first liquid L1. Furthermore, oil droplets D could be prepared with a relative error of about 1% to 6% or less in most of particle diameters.

Also, in the embodiment, since a specific gravity of the first liquid L1 is larger than a specific gravity of the second liquid L2, when the first liquid L1 is caused to flow into the second liquid L2, oil droplets D with a small relative error in particle diameter can be prepared without a problem that the first liquid L1 floats with respect to the second liquid L2 and spherical oil droplets D are not able to be stably formed.

(Second Step)

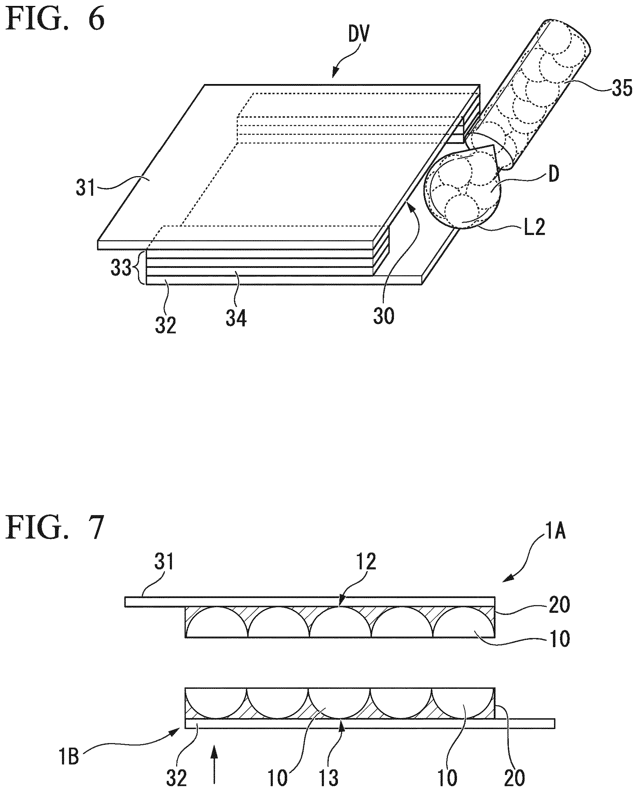

Next, a second step will be described. Fig. 6 is an external perspective view showing an example of a device DV used in the second step. The device DV includes the glass substrates 31 and 32 arranged parallel to each other to sandwich the gap 30 and a spacer 33 for defining a distance between the glass substrates 31 and 32. The spacer 33 is constituted of a plurality of stackable sheet members 34 arranged at both edges of the glass substrates 31 and 32.

In the second step, first, the number of stacked sheets of the sheet members 34 is adjusted in order to adjust a distance between the glass substrates 31 and 32 to a distance according to a particle diameter of the oil droplets D prepared in the first step. Specifically, for example, a plurality of fluororesin tapes having drip-proofing properties (liquid-tightness) each of which is with a thickness of 130 $\mu$m are prepared as the sheet members 34. Considering that a thickness may be increased by about 10 $\mu$m due to an influence of bubbles, when the plurality of fluororesin tapes are adhered to the glass substrates 31 and 32, a thickness of the gap 30 between the glass substrates 31 and 32 can be adjusted to $140 \times n$ ($\mu$m) by stacking n sheets of fluororesin tapes. Furthermore, the thickness of the gap 30 can also be adjusted to an arbitrary thickness using a fluororesin tape having a thickness other than 130 $\mu$m. Since four sheets of sheet members 34 are used in the example shown in Fig. 6, the thickness of the gap 30 is adjusted to about 560 $\mu$m.

When the thickness of the gap 30 is adjusted, the second liquid L2 containing the oil droplets D prepared in the first step is suctioned by a holding tool 35 such as a syringe and injected from the lower glass substrate 32 exposed upward into the gap 30. The second liquid L2 containing the oil droplets D is smoothly injected into the gap 30 using the capillary phenomenon.

Also in the second step, since a specific gravity of the oil droplets D (first liquid L1) is larger than a specific gravity of the second liquid L2, it is possible to prevent the oil droplets D injected into the gap 30 from floating with respect to the second liquid L2 and localizing upward in a thickness direction of the device DV.

(Third Step)

In a third step, irradiation with ultraviolet light as curing light is performed from outside of the glass substrates 31 and 32 through each of the glass substrates 31 and 32, for example, for 30 minutes. Thus, the second liquid L2 is cured by the irradiation of ultraviolet light UV and the base 20 made of a polyacrylamide that is an external phase is formed as a cured product containing the surfactant and the stabilizer is formed.

(Fourth Step)

Next, a fourth step will be described. In the fourth step, the oil droplets D remaining in the base 20 are removed to form the hole sections 10. The device DV in which the second liquid L2 is cured in the third step and the base 20 is formed is dried at 90°C for several hours and then immersed in acetone to be cleaned. Thus, the oil droplets D are removed as shown in Fig. 1 and the hole sections 10 arranged in a zigzag shape with regularity are formed as shown in Fig. 2. As described above, each of the diameters of the hole sections 10 has a size depending on the result obtained by selecting the first capillary device 40 or the second capillary device 50 and appropriately selecting the diameter of the nozzle 42, the diameter of the opening 49, and the flow rate of the first liquid L1.

Here, in the second step, there is no second liquid L2 or there is a minute amount of second liquid L2 at a portion at which the oil droplets D injected into the gap 30 between the glass substrates 31 and 32 are in contact with each other or a portion at which a distance between neighboring oil droplets D is the shortest. Thus, in the third step, these portions do not have the base 20 formed by curing the second liquid L2 or have a very small thickness. For this reason, through the cleaning in the fourth step, these portions do not have the base 20 formed therein and the neighboring hole sections 10 communicate with each other via the communication holes 11.

When the above-described cleaning is performed by, for example, ultrasonic cleaning (about one hour), the device DV is divided at a central portion at which the communication holes 11 are formed and which has low strength in a thickness direction as shown in Fig. 7. In this case, two porous films 1A and 1B in which hemispherical hole sections 10 are arranged in a state where their openings are exposed are prepared. Therefore, a porous film 1 having fully-spherical hole sections 10 formed therein and porous films 1A and 1B having hemispherical hole sections 10 formed therein can be selectively manufactured depending on the presence or absence of ultrasonic cleaning. As described above, in the porous films 1A and 1B, a problem such that the oil droplets D float with respect to the second liquid L2 and thus are localized upward when the specific gravity of the oil droplets D (first liquid L1) is smaller than the specific gravity of the second liquid L2 is prevented. Thus, the hole sections 10 are arranged in a uniform distribution without being localized upward.

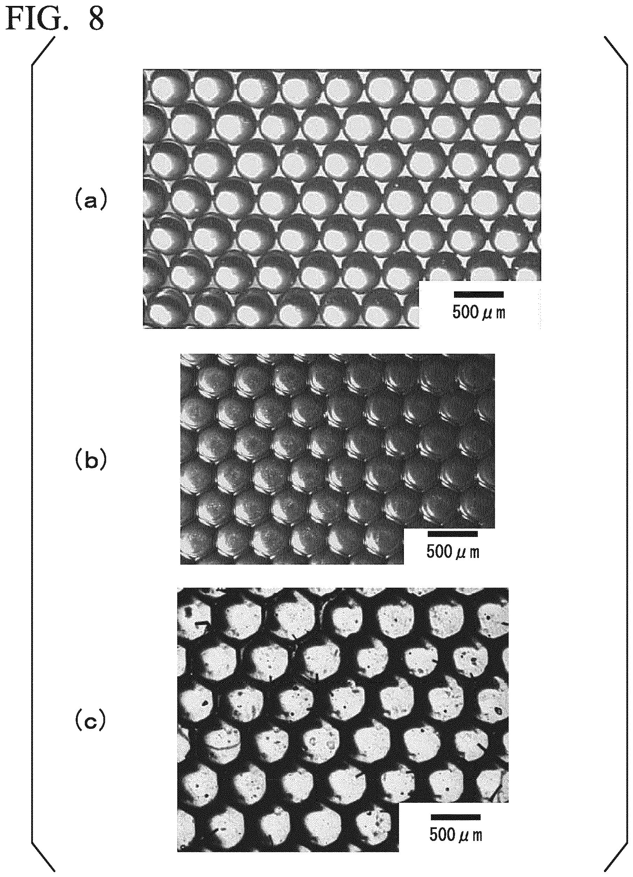

(a) of Fig. 8 shows a state in which oil droplets D are arranged in a zigzag shape before irradiation with ultraviolet light UV and (b) of Fig. 8 shows a state in which a second liquid L2 is hardened by irradiation with ultraviolet light UV. Furthermore, (c) of Fig. 8 shows a porous film 1A in which openings of hole sections 10 are exposed due to division.

Note that, when the porous films 1, 1A, and 1B are separated from the glass substrates 31 and 32, the device DV may be immersed alternately in cold water and hot water.

In the present embodiment, as described above, by using oil droplets D having a desired diameter and a photosetting second liquid L2 , a porous film having a hole diameter with a small variation of about 10 $\mu$m or more and 2000 $\mu$m or less can be manufactured at low cost. Furthermore, in the present embodiment, by selecting the first capillary device 40 or the second capillary device 50and appropriately selecting the diameter of the nozzle 42, the diameter of the opening 49, and the flow rate of the first liquid L1in the first step, an arbitrary hole diameter of 10 $\mu$m or more and 2000 $\mu$m or less can be easily selected and formed.

Also, since the specific gravity of the first liquid L1 is larger than the specific gravity of the second liquid L2 in the present embodiment, porous films 1, 1A, and 1B in which a relative error between particle diameters is small and hole sections 10 are arranged in a uniform distribution can be prepared without a problem such that the oil droplets D (first liquid L1) float with respect to the second liquid L2 and thus the spherical oil droplets D cannot be stably formed and the hole sections 10 are localized upward in a case in which the specific gravity of the first liquid L1 is smaller than the specific gravity of the second liquid L2.

Also, it is conceivable that for example, a gaseous phase such as bubbles may be used instead of a liquid phase when hole sections 10 are formed, but in this case, as described above, since a specific gravity of a gas is smaller than a specific gravity of a second liquid L2, there is concern that there may be a problem such as the hole sections 10 not being able to be stably formed or a problem of the hole sections 10 being arranged in a localized distribution. On the other hand, since the hole sections 10 are formed using the oil droplets D that are a liquid phase in the present embodiment, the specific gravity is easily adjusted as compared with the case of the gaseous phase, and porous films 1, 1A, and 1B in which a relative error between particle diameters is small, and hole sections 10 are arranged in a uniform distribution can be easily prepared.

It is also conceivable to use, for example, a solid phase instead of a liquid phase when hole sections 10 are formed. However, when spherical bodies to be arranged have a solid phase, the spherical bodies are rigid and when the spherical bodies are aggregated at arbitrary positions, there is no flexibility to rearrange these. For this reason, a problem that the hole sections 10 may not be able to be arranged uniformly is concerned.

Note that, when the hole diameter of the hole sections 10 is set to a value different from the average particle diameter shown in Fig. 5, for example, a constitution in which oil droplets D are formed by discharging the first liquid L1 by an inkjet method using a piezoelectric element such as a piezo element may be provided in addition to the method of continuously discharging the first liquid L1 from the nozzle 42 of the injection tube 41 as described above. When the oil droplets D are formed by an inkjet method, more minute oil droplets D can also be formed.

(Second Embodiment of Porous Film)

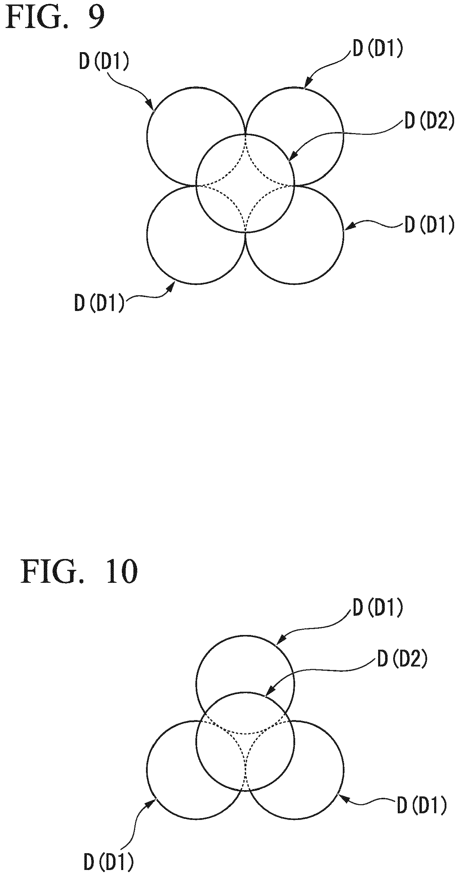

Next, a porous film according to a second embodiment will be described below with reference to Figs. 9 to 14. Although an exemplary example of a case in which the above porous film 1 is configured such that a layer of hole sections 10 is arranged in the thickness direction of the porous film 1 has been provided, an example in which a plurality of layers (here two layers) of hole sections 10 are arranged will be described in the second embodiment. In these drawings, constituent elements that are the same as constituent elements of the porous film 1 in the first embodiment shown in Figs. 1 to 8 will be denoted with the same reference numerals and description thereof will be omitted.

Figs. 9 and 10 are plan views showing a model of the oil droplets D (that is, the hole sections 10) arranged in two layers in the thickness direction in the above second step. Fig. 9 is a diagram showing a body-centered cubic structure in which an oil droplet D2 of a second layer is arranged in a gap formed by four oil droplets D1 of a first layer. Fig. 10 is a diagram showing a closed-packed structure (hexagonal close-packed structure) in which an oil droplet D2 of a second layer is arranged in a gap formed by three oil droplets D1 of a first layer.

When the oil droplets D are arranged in the body-centered cubic structure, a height of the two layers (thickness of a gap 30) is represented by the following Expression (1) if radii of the oil droplets D are assumed to be r.

[Math. 1]

$$\left(2+\sqrt{2}\right)r \qquad \cdots (1)$$

Also, when the oil droplets D are arranged in the closed-packed structure, a height of the two layers (thickness of a gap 30) is represented by the following Expression (2) if radii of the oil droplets D are assumed to be r.

[Math. 2]

$$\left(1+\frac{\sqrt{6}}{3}\right)2r \qquad \cdots (2)$$

Therefore, in the case in which the oil droplets D have a two-layer structure, in the above second step, the diameter of the hole sections 10 and the thickness of the gap 30 between the glass substrates 31 and 32 are adjusted to a thickness calculated by Expression (1) or Expression (2) depending on the structure. For example, if the radii of the hole sections 10 (droplets D) are 200 $\mu$m (diameter: 400 $\mu$m), in the case of the body-centered cubic structure, the thickness of the gap 30 is adjusted to 682 $\mu$m, and in the case of the closed-packed structure, the thickness of the gap 30 is adjusted to 726 μm. When the oil droplets D and the second liquid L1 are injected into the gap 30 whose thickness has been adjusted in the second step, the oil droplets D are arranged three-dimensionally in the body-centered cubic structure or the closed-packed structure in accordance with the thickness of the gap 30.

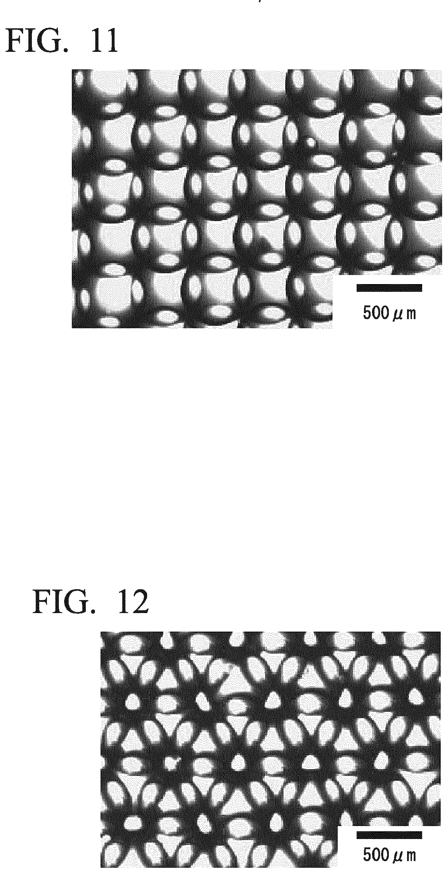

Fig. 11 is a diagram showing a state in which oil droplets D are arranged in a body-centered cubic structure. Fig. 12 is a diagram showing a state in which oil droplets D are arranged in a closed-packed structure.

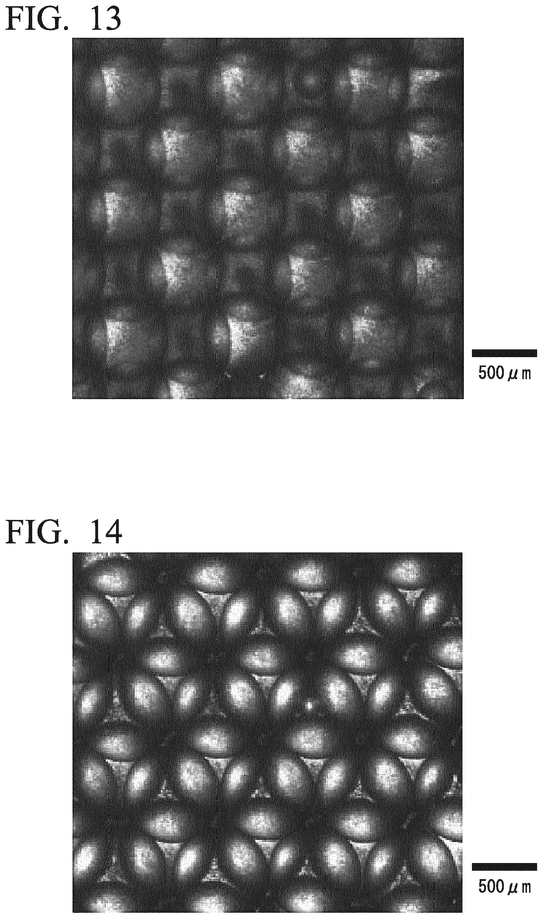

After that, when the above processes of the third step and the fourth step are sequentially performed, a porous film in which hole sections 10 with a small variation of a hole diameter are arranged in the body-centered cubic structure or the closed-packed structure can be manufactured. Fig. 13 is an enlarged view of the porous film 1 in which the hole sections 10 are arranged in the body-centered cubic structure. Fig. 14 is an enlarged view of the porous film 1 in which the hole sections 10 are arranged in the closed-packed structure.

If a porous film with a structure in which a plurality of layers of hole sections 10 are arranged is manufactured in this way, it is extremely difficult to perform manufacturing by switching between a body-centered cubic structure and a closed-packed structure when the hole sections 10 are formed in the gaseous phase as described above, but if the hole sections 10 are formed in a liquid phase like in the present embodiment, it is possible to perform manufacturing by easily switching between the body-centered cubic structure and the closed-packed structure in accordance with the thickness of the gap 30.

A constitution in which the hole sections 10 are arranged in two layers is exemplified in the second embodiment, but when the thickness of the gap 30 is set to a value depending on the body-centered cubic structure or the hexagonal close-packed structure, a porous film in which the hole sections 10 are formed in the body-centered cubic structure or the hexagonal close-packed structure over three layers or more can be manufactured.

(Third Embodiment of Porous Film)

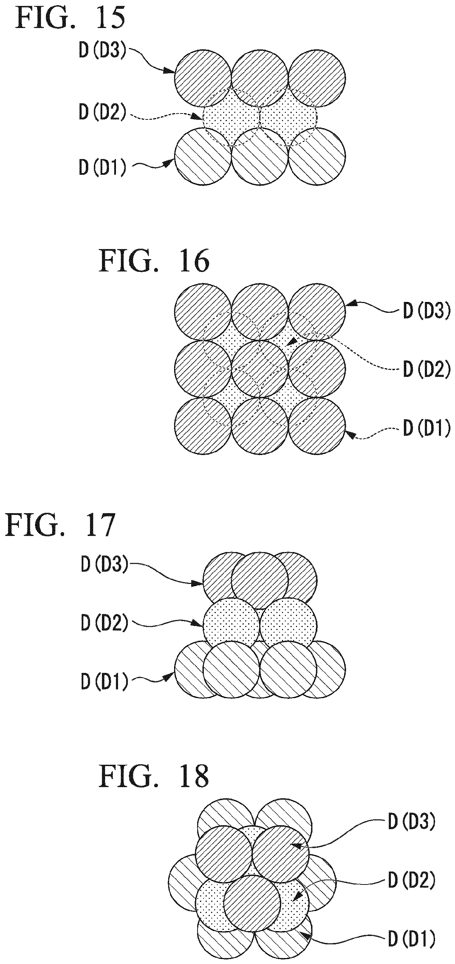

A porous film according to a third embodiment will be described below with reference to Figs. 15 to 18. A constitution in which the hole sections 10 are arranged in two layers in a thickness direction of the porous film 1 has been described in the second embodiment, but a constitution in which the hole sections 10 are arranged in three layers will be described in the third embodiment. In these drawings, constituent elements that are the same as the constituent elements of the porous film 1 in the second embodiment shown in Figs. 9 to 14 will be denoted with the same reference numerals and a description thereof will be omitted.

Fig. 15 is a front view of a model in which the oil droplets D (that is, hole sections 10) arranged in three layers in a thickness direction are arranged in a body-centered cubic structure in the above second step and Fig. 16 is a plan view viewed from a third layer side. As shown in Figs. 15 and 16, oil droplets D1 of a first layer and oil droplets D3 of a third layer are arranged to have a 3×3 lattice form having nine droplets and oil droplets D2 of a second layer are arranged to have a 2×2 lattice form having four droplets such that the oil droplets D2 of the second layer are located in gaps formed by four oil drops adjacent to each other in the first layer and the third layer.

When the oil droplets D (D1 to D3) are arranged in the body-centered cubic structure, a height of the three layers (thickness of the gap 30) is represented by the following Expression (3) if radii of the oil droplets D (D1 to D3) are assumed to be r.

[Math. 3]

$$2\left(1 + \sqrt{2}\right)r \qquad \cdot\ \cdot\ \cdot\ (3)$$

Fig. 17 is a front view of a model in which the oil droplets D (that is, the hole sections 10) arranged in the three layers in the thickness direction in the above second step are arranged in a face-centered cubic structure and Fig. 18 is a plan view viewed from the third layer side. As shown in Figs. 17 and 18, in a first layer, seven oil droplets D1 are arranged such that three of the seven oil droplets D1 are adjacent to and in contact with each other. In a second layer, each of three oil droplets D2 is arranged to be located in a gap formed by three oil droplets adjacent to each other in the first layer. In a third layer, each of three oil droplets D3 is arranged to be located in a gap formed by three oil droplets adjacent to each other in the second layer.

When the oil droplets D (D1 to D3) are arranged in the face-centered cubic structure, a height of the three layers (a thickness of a gap 30) is represented by the following Expression (4) if radii of the oil droplets D (D1 to D3) are assumed to be r.

[Math. 4]

$$2\left(1+\frac{2\sqrt{6}}{3}\right)r \qquad \cdots (4)$$

Thus, if the oil droplets D have a three layer structure, when a thickness of the gap 30 between the glass substrates 31 and 32 is adjusted to a thickness calculated by Expression (3) or (4) depending on a diameter of the hole sections 10 and their structure in the above second step, a porous film in which the three layers of hole sections 10 are arranged in the body-centered cubic structure or the face-centered cubic structure can be manufactured at low cost.

(Other Embodiments of Porous Film)

Although cases in which the diameters of the oil droplets D and the hole sections 10 are the same have been described in the above-described embodiments, when a plurality of types of oil droplets D with different diameters are prepared in the first step, a porous film 1 (or porous film 1A or 1B) having a plurality of types of hole sections 10 with different diameters can also be manufactured. A plurality of types of oil droplets D with different diameters can be prepared by, for example, selecting the above-described first capillary device 40 or second capillary device 50 and changing at least one of a diameter of the nozzle 42, a diameter of the opening 49, and a flow rate of the first liquid L1. Particularly, a plurality of types of oil droplets D with different diameters can be continuously formed by selecting a method of changing a flow rate of the first liquid L1.

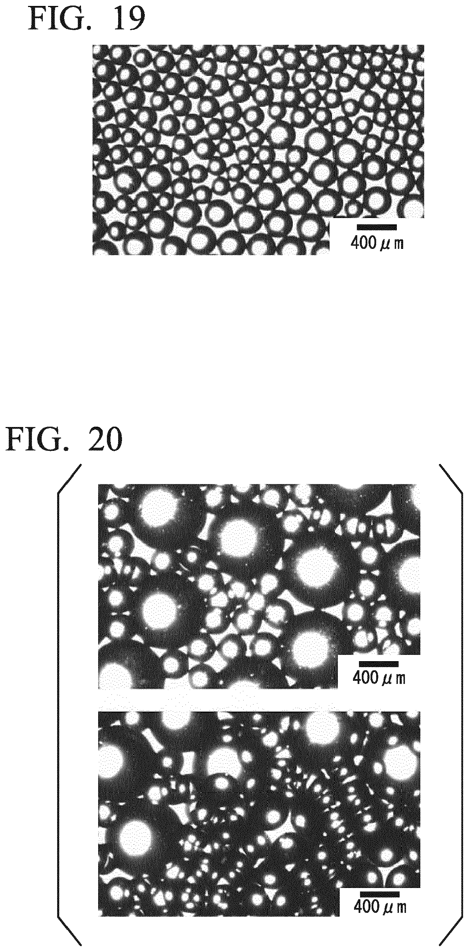

Fig. 19 is a diagram showing a state of arrangement in which there are two kinds of oil droplets D with a diameter ratio of about 2:3 before irradiation with ultraviolet rays UV is performed. Fig. 20 is a diagram showing a state of arrangement in which there are two kinds of oil droplets D with a diameter of about 1:2 before irradiation with ultraviolet rays UV is performed. As shown in Fig. 19, in the case of oil droplets D with a relatively small diameter ratio, the oil droplets D are arranged at positions depending on an order in which the oil droplets D have been formed. As shown in Fig. 20, in the case of oil droplets D with a relatively large diameter ratio, an arrangement in which oil droplets D with a small diameter are induced due to the arrangement of the oil droplets D with the large diameter is obtained. By doing these, when a plurality of types of oil droplets D with different diameters are prepared, a plurality of types of hole sections 10 with different diameters can be easily formed in a state in which there is little variation for each hole diameter.

(Application Example of Porous Film)

An application example of the above porous film will be described below.

[Microlens array]

A porous film 1 according to the present embodiment can be applied to a microlens array.

It is expected that a microlens array will be able to be applied to control of optical properties such as light flux, polarization of light, and a wavelength or preparation of a three-dimensional stereoscopic image by interference of lights from a large number of lens bodies of a microlens array in which micro lenses are arranged regularly. The porous film 1 according to the present embodiment functions as a microlens array when lens bodies are arranged in hole sections 10. Examples of the lens bodies can include a cholesteric liquid crystal microcapsule which is an omni-directional laser oscillator.

A cholesteric liquid crystal is one of liquid crystal materials and has a feature, i.e., "bistability" which means that it can be stable in a state in which it transmits light (focal conic state) and a state in which it reflects light (planar state) without applying electric power. A cholesteric liquid crystal is prepared by adding an additive called a chiral agent to a nematic liquid crystal so as to have optical rotary power. Furthermore, a cholesteric liquid crystal is referred to as a chiral nematic liquid crystal (CN liquid crystal) in some cases. Since a cholesteric liquid crystal has memory characteristics, a display does not disappear even if electric power is turned off, low electric power consumption becomes possible, and the cholesteric liquid crystal can be applied to electronic paper display technology.

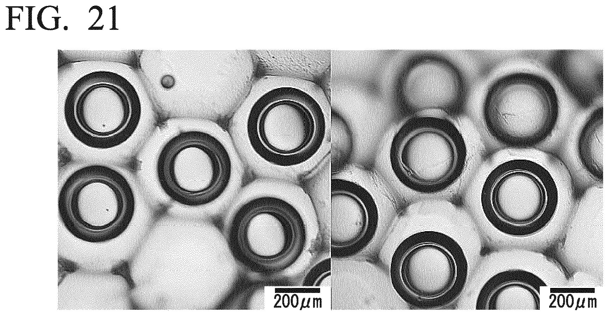

As shown in Fig. 21, cholesteric liquid crystal microcapsules have been captured in hole sections 10 with a larger diameter than a diameter of the cholesteric liquid crystal microcapsules using a porous film 1 having the hole sections 10. Since each of the captured liquid crystal microcapsules acts as a liquid crystal lens, the liquid crystal microcapsule functions as a microlens array whose refractive index can be controlled by an external field. Since the above-described porous film 1 is provided in the present embodiment, a microlens array having a lens body of about 10 $\mu$m or more and 2000 $\mu$m or less can be manufactured at low cost.

[Microreactor]

The porous film 1 according to the present embodiment can be applied to a microreactor.

A microreactor uses a microspace as a reaction field, and in the porous film 1 according to the present embodiment, each of the hole sections 10 functions as a reaction field. Application of a microreactor to a heterogeneous catalytic reaction can be conceived because a specific surface area per volume of the microreactor is large. For this reason, the porous film 1 functions as a microreactor by carrying a catalyst on the porous film 1.

In addition, the reaction in the reaction field can be controlled by controlling a lattice structure or a micro hole diameter of the porous film 1. Since the above-described porous film 1 is provided in the present embodiment, a microreactor having a reaction field of about 10 $\mu$m or more and 2000 $\mu$m or less can be manufactured at low cost.

[Bio-device]

The porous film 1 according to the present embodiment can be applied to a bio-device.

Examples of the bio-device include devices serving as scaffolds for cell patterning and devices serving as scaffolds for growth of cell or the like. In order to use cell patterning as, for example, a bio-sensor, it is necessary to control a position at which a cell culture is performed. Cell patterning can be realized by preparing the porous film 1 according to the present embodiment using substances which are not adherent to cells or subjecting the porous film 1 to surface treatment with substances which are not adherent to cells, adhering the porous film 1 on a substrate on which cells are to be cultured, adhering cells on the substrate, and separating the porous film 1. Since this cell patterning uses the porous film 1 according to the present embodiment, a patterning operation of a top-down substrate can be shortened and significant cost reduction can be achieved.

Many conventional porous films are two-dimensional films and two-dimensional growth in cell growth or blood vessel growth has been researched, but when neurospheres are attached to a surface of the porous film 1 using the porous film 1 according to the present embodiment in which the hole sections 10 are formed three-dimensionally and a growth process is observed, three-dimensional growth regarded to be indispensible when application thereof to an actual brain or a human body is considered can be researched at low cost. In addition, different growth processes in the porous film 1 with different lattice structures can also be observed, which can contribute to basic research.

[Template]

The porous film 1 according to the present embodiment can be applied to a template.

In the porous film 1, the base 20 serves as a mold and a material disposed in each of the hole sections 10 can be molded as a template. In this case, the number of the oil droplets D injected into the gaps 30 between the glass substrates 31 and 32 in the second step may be set not to be dense. With such a constitution, neighboring hole sections 10 can be formed independently without communicating with each other and hole sections 10 in which only portions thereof in contact with the glass substrates 31 and 32 are open can be formed.

While preferred embodiments associated with the present invention have been described above with reference to the accompanying drawings, it goes without saying that the present invention is not limited to these examples. The forms, combinations, or the like of the constituent elements shown in the above-described examples are merely examples and various modifications are possible based on design requirements or the like without departing from the gist of the present invention.

For example, examples of a constitution in which the first liquid L1 is the oil phase and the second liquid L2 is the aqueous phase have been provided in the above embodiments, but the present invention is not limited thereto and a constitution in which the first liquid L1 is an aqueous phase and the second liquid L2 is an oil phase may be provided. In this case, examples of the first liquid L1 which is the aqueous phase include deionized water and examples of the second liquid L2 which is the oil phase can include styrene monomers (84.2 % by weight), divinylbenzene (9.8 % by weight; a crosslinking agent), PLURONIC P123 (registered trademark) (1.1 % by weight; a surfactant), and IRGACURE (registered trademark) TPO (4.9 % by weight; a photopolymerization initiator).

An example of a method for manufacturing a porous film using thermal polymerization when the first liquid L1 is an aqueous phase and the second liquid L2 is an oil phase will be shown.

1) A solution was degassed for one hour in the above first step and then purged with nitrogen by bubbling for one hour to prepare a W/O type emulsion. At this time, the composition of the oil phase was 0.92 % by weight of a surfactant P123, 84.57 % by weight of a styrene monomer (from which a polymerization inhibitor had been removed), 8.66 % by weight of divinylbenzene (from which a polymerization inhibitor had been removed), and 5.84% by weight of a thermal polymerization initiator AIBN, and the aqueous phase was deionized water.

2) A porous film was formed by curing at room temperature using the above W/O type emulsion.

3) In order to minimize volatilization of the styrene monomer, a glass cell having the W/O type emulsion sealed therein was put in what is filled with the styrene monomers and was left to stand at room temperature.

By adopting such a method, a porous film can be manufactured by thermal polymerization.

An example of a method for manufacturing a porous film through photopolymerization when the first liquid L1 is an aqueous phase and the second liquid L2 is an oil phase will be shown below.

1) In the same manner as described above, a W/O type emulsion was prepared in the first step. At this time, the oil phase had a composition of 1.40 % by weight of a surfactant P123, 77.74 % by weight of toluene, and 20.85 % by weight of an acrylic curable resin (UNISOLAR (registered trademark), manufactured by Unitec Co., Ltd) and the aqueous phase was deionized water.

2) A porous film was formed by curing the above-described W/O type emulsion through irradiation with ultraviolet light UV.

By adopting such a method, a porous film can be manufactured by photopolymerization.

Although an exemplary example of a constitution in which the second liquid L is cured by imparting light energy to the second liquid L has been provided in the above embodiments, the present invention is not limited thereto. In addition, a constitution in which the second liquid L includes an initiator for curing the second liquid L when thermal energy is imparted, and the base 20 is formed by applying the thermal energy to the second liquid L in the third step so as to cure the second liquid L may be adopted.

As a method for forming the base 20, a constitution in which the base 20 is formed by using a curing agent which cures due to change in pH instead of the curing agent which cures by imparting light energy or thermal energy and curing the second liquid L by changing a pH of the second liquid L may be adopted i.

To be specific, the second liquid L can be cured using a redox initiator such as ammonium persulfate, hydrogen peroxide, and diisopropylpercarbonate as a curing agent which cures due to change in pH and tetraethylenediamine, triethylamine, triethanolamine, or the like as a material changing a pH of the second liquid L.

Although a case in which the hole diameter of the hole sections 10 is a predetermined diameter of 10 $\mu$m or more and 2000 $\mu$m or less has been exemplified in the above-described embodiments, in order to more stably form the hole sections 10, it is desirable that a hole diameter of the hole sections 10 be 250 $\mu$m or more and 2000 $\mu$m or less.

Although a constitution in which the plurality of hole sections 10 are arranged in a body-centered cubic structure or a hexagonal close-packed structure has been described in the above-described embodiments, a porous film in which the hole sections 10 are arranged in a face-centered cubic structure can be manufactured in accordance with the present invention in addition to these.

[List of References]

1 Porous film
10 Hole section
11 Communication hole
20 Base
30 Gap
31, 32 Glass substrate (substrate)
41 Injection tube (first tube)
41a Flow path
42 Nozzle
46, 47 Recovery tube (second tube)
D Oil droplet (droplet)
L1 First liquid
L2 Second liquid
UV Ultraviolet light (light)

**Claims**

1. A method for manufacturing a porous film comprising:

a first step of preparing droplets which are formed from a first liquid into spheres with a predetermined diameter of 10 μm or more and 2000 μm or less and a second liquid which includes a curing agent which cures by imparting energy or a curing agent which cures due to change in pH and includes the droplets dispersed therein; a second step of injecting the droplets and the second liquid into a gap between a pair of substrates; a third step of curing the second liquid to form a base; and a fourth step of removing the droplets in the base to form hole sections.

**2.** The method for manufacturing a porous film according to claim 1, wherein the second liquid includes a surfactant and a stabilizer.

**3.** The method for manufacturing a porous film according to claim 1 or 2, wherein the energy is light or heat.

**4.** The method for manufacturing a porous film according to any one of claims 1 to 3, wherein a specific gravity of the first liquid is larger than a specific gravity of the second liquid.

**5.** The method for manufacturing a porous film according to any one of claims 1 to 4, wherein the distance between the pair of substrates is adjusted so that the droplets are arranged in one layer in the gap.

**6.** The method for manufacturing a porous film according to any one of claims 1 to 4, wherein a distance between the pair of substrates is adjusted so that the droplets are arranged in a plurality of layers in the gap.

**7.** The method for manufacturing a porous film according to claim 6, wherein the droplets are arranged in a body-centered cubic structure.

**8.** The method for manufacturing a porous film according to claim 6, wherein the droplets are arranged in a hexagonal close-packed structure.

**9.** The method for manufacturing a porous film according to claim 6, wherein the droplets are arranged in a face-centered cubic structure.

**10.** The method for manufacturing a porous film according to any one of claims 1 to 9, wherein, in the first step, droplets of a plurality of types with different diameters are prepared.

**11.** The method for manufacturing a porous film according to any one of claims 1 to 10, wherein the first step includes a step of causing the first liquid to flow into a flow path of a first tube and causing the droplets of the first liquid to flow from a nozzle of the first tube into the second liquid flowing through a flow path of a second tube.

**12.** The method for manufacturing a porous film according to claim 11, wherein at least one of a relative rate of the first liquid flowing through the first tube with respect to a rate of the second liquid flowing through the second tube and a diameter of the nozzle is adjusted depending on a diameter of the droplets to be prepared.

**13.** The method for manufacturing a porous film according to any one of claims 1 to 12, wherein at least one of the first liquid and the second liquid has an oil phase and the other thereof has an aqueous phase.

**14.** The method for manufacturing a porous film according to any one of claims 1 to 13, wherein the step of removing the droplets includes a step of cleaning the droplets.

**15.** The method for manufacturing a porous film according to any one of claims 1 to 14, wherein the droplets are formed with a predetermined diameter of 250 μm or more and 2000 μm or less.

**16.** A porous film comprising:

a plurality of hole sections formed in a spherical shape with a predetermined diameter of 10 μm or more and 2000 μm or less; and
a base including a curing agent cured by imparting energy or a curing agent cured due to change in pH, wherein the plurality of hole sections are arranged with a predetermined size and a relative error in a diameter thereof is 6% or less.

**17.** The porous film according to claim 16, wherein the base includes a surfactant and a stabilizer.

**18.** The porous film according to claim 16 or 17, wherein the hole sections arranged to be adjacent to each other communicate with each other via communication holes.

**19.** The porous film according to any one of claims 16 to 18, wherein the plurality of hole sections are arranged in a body-centered cubic structure.

**20.** The porous film according to any one of claims 16 to 18, wherein the plurality of hole sections are arranged in a hexagonal close-packed structure.

**21.** The porous film according to any one of claims 16 to 18, wherein the plurality of hole sections are arranged in a face-centered cubic structure.

**22.** The porous film according to any one of claims 16 to 21, wherein the plurality of hole sections include a plurality of first hole sections formed with a first diameter and a plurality of second hole sections formed with a second diameter different from the first diameter, and
the first hole sections and the second hole sections are arranged in a predetermined regular manner and relative errors in diameters thereof are 6% or less.

**23.** The porous film according to any one of claims 16 to 22, wherein the hole sections are formed with a predetermined diameter of 250 $\mu$m or more and 2000 $\mu$m or less.

**24.** A microlens array comprising:

the porous film according to any one of claims 16 to 23; and
lens bodies arranged in the hole sections.

**25.** A microreactor comprising:
the porous film according to any one of claims 16 to 23.

**26.** A bio-device comprising:
the porous film according to any one of claims 16 to 23.

FIG. 1

FIG. 2

FIG. 3

FIG. 4

**(a)**

| FLOW RATE(ml/h) | AVERAGE PARTICLE DIAMETER($\mu$m) | RELATIVE ERROR(%) |
|---|---|---|
| 0.1 | 189 | 3.22 |
| 0.6 | 214 | 3.03 |
| 1.1 | 230 | 3.54 |
| 2.1 | 426 | 4.04 |
| 2.6 | 449 | 2.63 |
| 3.1 | 483 | 2.68 |
| 7.1 | 634 | 1.21 |

**(b)**

| FLOW RATE(ml/h) | AVERAGE PARTICLE DIAMETER($\mu$m) | RELATIVE ERROR(%) |
|---|---|---|
| 0.6 | 340 | 1.61 |
| 1.1 | 347 | 5.95 |
| 1.6 | 372 | 2.33 |
| 2.1 | 396 | 1.48 |
| 2.6 | 401 | 1.32 |
| 3.1 | 414 | 3.19 |
| 7.1 | 530 | 1.49 |

**(c)**

| FLOW RATE(ml/h) | AVERAGE PARTICLE DIAMETER($\mu$m) | RELATIVE ERROR(%) |
|---|---|---|
| 0.1 | 487 | 1.79 |
| 0.6 | 566 | 1.58 |
| 1.1 | 628 | 1.72 |
| 1.6 | 702 | 1.09 |
| 2.1 | 824 | 5.49 |
| 2.6 | 849 | 2.63 |
| 3.1 | 898 | 2.71 |
| 7.1 | 1108 | 4.57 |

**(d)**

| FLOW RATE(ml/h) | AVERAGE PARTICLE DIAMETER($\mu$m) | RELATIVE ERROR(%) |
|---|---|---|
| 0.6 | 920 | 1.90 |
| 1.1 | 932 | 2.18 |
| 1.6 | 1000 | 0.76 |
| 2.1 | 1021 | 1.52 |
| 2.6 | 1046 | 2.65 |
| 3.1 | 1113 | 3.57 |
| 7.1 | 1272 | 3.28 |

**(e)**

| FLOW RATE(ml/h) | AVERAGE PARTICLE DIAMETER($\mu$m) | RELATIVE ERROR(%) |
|---|---|---|
| 2 | 110 | 0.58 |
| 4 | 96 | 0.66 |
| 6 | 84 | 1.18 |
| 8 | 71 | 1.07 |
| 10 | 68 | 0.98 |
| 12 | 64 | 1.36 |

FIG. 5

FIG. 6

FIG. 7

FIG. 8

(a)

500 μm

(b)

500 μm

(c)

500 μm

FIG. 9

D (D1)

D (D1)

D (D2)

D (D1)

D (D1)

FIG. 10

D (D1)

D (D2)

D (D1)

D (D1)

FIG. 11

500 μm

FIG. 12

500 μm

FIG. 13

500 μm

FIG. 14

500 μm

FIG. 15

D (D3)

D (D2)

D (D1)

FIG. 16

D (D3)

D (D2)

D (D1)

FIG. 17

D (D3)

D (D2)

D (D1)

FIG. 18

D (D3)

D (D2)

D (D1)

FIG. 19

FIG. 20

FIG. 21

## INTERNATIONAL SEARCH REPORT

| International application No. |
|---|
| PCT/JP2016/086905 |

A. CLASSIFICATION OF SUBJECT MATTER
*C08J9/28*(2006.01)i, *B01J19/00*(2006.01)i, *B29C39/24*(2006.01)i, *C12M3/00*
(2006.01)i, *G02B3/00*(2006.01)i, *B29K105/04*(2006.01)n

According to International Patent Classification (IPC) or to both national classification and IPC

B. FIELDS SEARCHED

Minimum documentation searched (classification system followed by classification symbols)
C08J9/00-42, B29C39/00-44

Documentation searched other than minimum documentation to the extent that such documents are included in the fields searched
Jitsuyo Shinan Koho          1922-1996   Jitsuyo Shinan Toroku Koho   1996-2017
Kokai Jitsuyo Shinan Koho    1971-2017   Toroku Jitsuyo Shinan Koho   1994-2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2000-355604 A  (Nippon Shokubai Co., Ltd.),<br>26 December 2000 (26.12.2000),<br>claims; paragraphs [0050] to [0062], [0069] to<br>[0071], [0075] to [0077], [0080], [0081];<br>examples 15, 16, 18; table 3<br>& US 6166097 A<br>claims; examples 15 to 18; table 3<br>& EP 1045001 A2          & CN 1270177 A | 1-4,13,14<br>5-12,15-26 |

| ☒ | Further documents are listed in the continuation of Box C. | ☐ | See patent family annex. |
|---|---|---|---|

*   Special categories of cited documents:
"A"  document defining the general state of the art which is not considered   to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search<br>    03 March 2017 (03.03.17) | Date of mailing of the international search report<br>    14 March 2017 (14.03.17) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |

Form PCT/ISA/210 (second sheet) (January 2015)

**INTERNATIONAL SEARCH REPORT**

International application No.

PCT/JP2016/086905

C (Continuation).    DOCUMENTS CONSIDERED TO BE RELEVANT

| Category* | Citation of document, with indication, where appropriate, of the relevant passages | Relevant to claim No. |
|---|---|---|
| X<br>A | JP 2002-527335 A  (Allied-Signal Inc.),<br>27 August 2002 (27.08.2002),<br>claims; paragraphs [0014], [0022], [0023],<br>[0025], [0063]; examples; fig. 3, 4, 11<br>& US 2001/0019037 A1<br>claims; paragraphs [0003], [0057], [0058],<br>[0100], [0101]; examples; fig. 3, 4, 11<br>& WO 2000/021905 A1      & EP 1121334 A1<br>& CA 2343915 A | 16,18-23,25<br>1-15,17,24,<br>26 |
| X<br><br>A | JP 2013-504669 A  (Spheritech Ltd.),<br>07 February 2013 (07.02.2013),<br>claims; paragraphs [0001], [0018], [0024],<br>[0032], [0033], [0046], [0074]; examples<br>& US 2012/0309053 A1<br>claims; paragraphs [0001], [0018], [0025],<br>[0035], [0036], [0049], [0081]; examples<br>& GB 2473814 A          & WO 2011/032704 A2<br>& EP 2478045 A1          & CN 102630240 A | 16,18-23,25,<br>26<br>1-15,17,24 |
| A | JP 2012-509960 A  (Corning Inc.),<br>26 April 2012 (26.04.2012),<br>entire text<br>& US 2010/0129912 A1     & WO 2010/059902 A2<br>& EP 2361277 A1          & CN 102292384 A | 1-26 |
| A | JP 2015-527469 A  (Wacker Chemie AG.),<br>17 September 2015 (17.09.2015),<br>entire text<br>& US 2015/0218334 A1     & WO 2014/037197 A1<br>& EP 2892637 A1          & DE 102012215881 A<br>& TW 201410314 A         & KR 10-2015-0044962 A<br>& CN 104602795 A | 1-26 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015243016 A
- JP 2016142659 A

- JP 2014156526 A

**Non-patent literature cited in the description**

- **H. J. LEE et al.** *Acta Biomaterialia,* 2011, vol. 7, 1281-1889
- **P. YILGOR et al.** *J. Tissue Eng. Regen. Med.,* 2013, vol. 7, 687-696

- **H. YABU et al.** *Macromolecules,* 2011, vol. 44, 5868-5873